(19) Europäisches Patentamt
European Patent Office
Office européen des brevets

(11) **EP 1 975 156 B1**

(12) **EUROPEAN PATENT SPECIFICATION**

(45) Date of publication and mention
of the grant of the patent:
**02.03.2011 Bulletin 2011/09**

(51) Int Cl.:
**C07D 201/12** *(2006.01)* **C07D 223/04** *(2006.01)*
**C08J 11/16** *(2006.01)* **C08J 11/20** *(2006.01)*

(21) Application number: **06823070.5**

(22) Date of filing: **07.11.2006**

(86) International application number:
**PCT/JP2006/322157**

(87) International publication number:
**WO 2007/060828 (31.05.2007 Gazette 2007/22)**

(54) **METHOD FOR DEPOLYMERIZING POLYAMIDE AND METHOD FOR PRODUCING MONOMER OF POLYAMIDE**

VERFAHREN ZUR DEPOLYMERISIERUNG EINES POLYAMIDS UND VERFAHREN ZUR HERSTELLUNG EINES MONOMERS EINES POLYAMIDS

PROCÉDÉ SERVANT À DÉPOLYMÉRISER UN POLYAMIDE ET PROCÉDÉ SERVANT À PRODUIRE UN MONOMÈRE DE POLYAMIDE

(84) Designated Contracting States:
**DE ES**

(30) Priority: **25.11.2005 JP 2005339752**

(43) Date of publication of application:
**01.10.2008 Bulletin 2008/40**

(73) Proprietor: **Ube Industries, Ltd.**
**Ube-shi,**
**Yamaguchi-ken 755-8633 (JP)**

(72) Inventors:
• **KAISO, Kouji**
**Ube-shi, Yamaguchi 7558633 (JP)**
• **SUGIMOTO, Tsunemi**
**Ube-shi, Yamaguchi 7558633 (JP)**

(74) Representative: **Bublak, Wolfgang**
**Bardehle Pagenberg**
**Postfach 86 06 20**
**81633 München (DE)**

(56) References cited:
**WO-A-02/16345** **JP-A- 01 071 854**
**JP-A- 10 509 965** **JP-A- 2000 191 638**
**JP-B1- 46 031 541**

• **DATABASE WPI Week 200227 Thomson Scientific, London, GB; AN 2002-210745 XP002519336 & JP 2001 316479 A (DAIA BOND KOGYO KK) 13 November 2001 (2001-11-13)**

**Description**

TECHNICAL FIELD

The present invention describes a polyamide depolymerization method capable of depolymerizing a polyamide to obtain a monomer at a higher yield. It also describes a method for producing a monomer of polyamide utilizing the depolymerization method.

BACKGROUND ART

Polyamide products, typically nylon 6 obtained through polymerization of caprolactam, have been utilized in large quantities as nylon fibers, films, and engineering plastics in various fields. Used polyamide products are used in landfill or burned as wastes. From the viewpoint of the environmental protection and the effective utilization of resources in recent years, various methods of recycling polyamide products have been studied.

For example, Patent Document 1 describes a method of depolymerizing a polyamide in water in the presence of a nitrogenous compound. It may cause a problem, however, associated with corrosion of a metal device in a relatively short period if a large quantity of high-temperature, high-pressure water is used. It also consumes a large quantity of energy on separation of a large quantity of water after a depolymerization reaction. It further requires advanced wastewater processing equipment in disposing a large quantity of water.

Patent Document 1: JP 8-301843A

DISCLOSURE OF THE INVENTION

PROBLEM TO BE SOLVED BY THE INVENTION

The present invention solves the above problem and has an object to provide an industrially advantageous method for depolymerizing Nylon 6 to obtain caprolactam easily and efficiently.

MEANS TO SOLVE THE PROBLEM

The inventors have eagerly studied the conditions for depolymerizing polyamides. As a result, they found that the recovery percentage of a monomer in a hydrocarbon solvent is improved greatly compared with the case of water and reached completion of the invention. Namely, the present invention describes a polyamide depolymerizing method for depolymerizing a polyamide to obtain a monomer, comprising performing a depolymerization reaction in the presence of a hydrocarbon solvent. The present invention also describes a polyamide monomer producing method, comprising subjecting a polyamide to a depolymerization reaction in the presence of a hydrocarbon solvent to obtain a monomer of the polyamide.

EFFECT OF THE INVENTION

In accordance with the present invention, the efficiency percentage of a monomer can be improved greatly. In addition, the use of a hydrocarbon-based organic solvent can prevent corrosion of the device. Further, the hydrocarbon solvent has a smaller latent heat of vaporization and accordingly the energy cost for recovery of the hydrocarbon solvent can be reduced. The hydrocarbon solvent can be used repeatedly and utilized effectively as a fuel on disposition. This makes it possible to industrially achieve material recycling of polyamide products, which have been used in landfill or burned in the art.

THE BEST MODE FOR CARRYING OUT THE INVENTION

The present invention will now be described in detail.
A polyamide described in the present invention is a polymer having two or more amide (-C(O)NH-) bonds. More specifically, it is a polymer having a diamine and a dicarboxylic acid, chained by an amide bond. Alternatively, it is a chain polymer, such as caprolactam, obtained through ring-opening polymerization of a monomer having an amino group and a carboxyl group, cyclized through dehydration condensation, in a molecule. A polyamide is not particularly restricted on the polymerization degree and may be a lower polymer or oligomer. Examples of the oligomer include chain forms (from a dimer to around a heptamer of aminocaproic acid) and ring forms (from a dimer to around a monomer. A polyamide may include one type or two or more types mixed. For example, nylon 6, nylon 66, nylon 11 and nylon 12 are listed. Nylon 6 is preferably used. Specific examples include: a waste of nylon 6 fiber carpets; out-of-tolerance products resulted from switching between product grades during continuous polymerization of caprolactam to produce nylon 6; residues containing an oligomer resulted from removal of water from cleaning water after hot water cleaning of polymers; and distillation residues resulted from continuous distillation of caprolactam in the step of producing caprolactam.

The following description is given to the hydrocarbon solvent. Examples of the hydrocarbon solvent usable in the present invention may be aliphatic and aromatic without distinction. Specific examples include: aliphatic hydrocarbons such as n-hexane, n-heptane, and cyclohexane; and aromatic hydrocarbons such as benzene and toluene. Of those, toluene is preferably used.

During a depolymerization reaction of a polyamide in the presence of a hydrocarbon solvent, the depolymerization reaction may be performed in the presence of water together with the hydrocarbon solvent. This is effective to reduce the reaction time required for depolymerization. In this case, the ratio of water relative to the total mass of the hydrocarbon solvent and water is above 0 and not more than 30 mass %, preferably not less than 1 mass % and not more than 20 mass %. If the ratio of water relative to the total mass of the hydrocarbon solvent and water is more than 30 mass %, a metal device may be easily corroded in a relatively short period undesirably.

The ratio of the polyamide relative to the total mass of the polyamide, hydrocarbon solvent and water is above 0 and not more than 50 mass %, preferably not less than 10 mass % and not more than 40 mass %.

The depolymerization reaction of the Nylon 6 in the present invention can use an acidic/alkaline catalyst. Without the use of any catalyst, however, a higher recovery percentage of monomer can be achieved. This eliminates the need for catalyst supply equipment, simplifies the depolymerization reaction system, and eliminates the need for the catalyst removal/recovery step. Accordingly, it can be an industrially excellent process.

The depolymerization reaction is performed at a temperature not lower than 300 °C and not higher than 420 °C, preferably not lower than 300 °C and not higher than 400 °C, particularly preferably not lower than 350 °C and not higher than 370 °C. A temperature below 300 °C is not preferable because the depolymerization reaction of a polyamide can not proceed sufficiently. On the other hand, a temperature above 420 °C is not preferable because the depolymerized monomer is further thermally decomposed, thereby lowering the recovery percentage.

The time for the depolymerization reaction is not less than 5 minutes and not more than 4 hours, preferably not less than 1 hour and not more than 2 hours. This makes it possible to recovery the monomer at a higher recovery percentage than the prior art.

From the treatment liquid after the depolymerization reaction, the hydrocarbon solvent is recovered at a hydrocarbon solvent recovery step. Examples of hydrocarbon solvent recovery means include a single distiller; a flash separator such as a flash drum; and a (reduce-pressure) distiller such as a distilling column. If the depolymerization reaction is performed in a hermetic state, the interior of the reactor is kept in a high-temperature, high-pressure state. Accordingly, after the reaction, a pressure regulation valve may be used to reduce the pressure inside the reactor such that the temperature arises above the boiling point of the hydrocarbon solvent. This simple method is applied to flash the hydrocarbon solvent to be recovered. This makes it possible to reduce the energy cost. The flashed hydrocarbon solvent is condensed in a condenser. The condensed hydrocarbon solvent is reused for a depolymerization reaction again.

From the residue after the recovery of the hydrocarbon solvent, a monomer is recovered at a monomer recovery step. Examples of monomer recovery means include a single distiller; a (reduce-pressure) distiller such as a distilling column; and a thin-film evaporator. Of those, the thin-film evaporator is preferably used to prevent thermal deterioration of the monomer. For example, if caprolactam resulted from the depolymerization of nylon 6 is processed in the thin-film evaporator, then the distillation temperature is kept at 80-120 °C and the pressure in the column top is kept at 0.05-2.66 kPaA (where A indicates the absolute pressure). The recovered monomer can be supplied again for the production of a polyamide, as it is, or after further purified by finely distillation, recrystallization or sublimation.

On the other hand, the residue containing non-reacted substances not depolymerized after the recovery of the monomer at the monomer recovery step is supplied again to the depolymerization reactor, thereby further improving the recovery percentage of the monomer.

EXAMPLES

The present invention is described in further detail with examples. The present invention is not limited to the following examples.

(Example 1)

As the reactor, a SUS316 (stainless steel) reaction tube having a volume of 10 mL was used. Into the reaction tube, 0.3 g of nylon 6 (Nylon Chip 1022B from Ube Industries, Ltd.) and 4 g of toluene (for organic synthesis (dehydrated) from Wako Pure Chemical Industries, Ltd., with 10 ppm of water) were added. Next, the air in the headspace in the reaction tube was replaced with argon and then the reaction tube was sealed. The sealed reaction tube was left stationary in an electrical furnace heated at 370 °C and subjected to heating for 6 hours. The pressure inside the reaction tube at this time was 7.6 MPaG (where G indicates gauge pressure). After heating, the reaction tube was extracted from the electrical furnace and the reaction tube was immersed into water and cooled down to the room temperature. The content was extracted from the cooled reaction tube and ε-caprolactam contained therein was quantified.

The quantification of ε-caprolactam was performed using a gas chromatography (GC-14B from Shimadzu Corporation). The recovery percentage of ε-caprolactam was calculated in accordance with the following equation. The result is shown in Table 1. The recovery percentage of caprolactam was 74.1 weight %. This indicates that the recovery percentage is further improved compared with the case of water in Comparative examples 1 and 2 shown below. The interior of the reactor after the reaction kept the luster of stainless steel when it was visually confirmed.

[Recovery percentage of ε-caprolactam (mass %)]

$$= [(\varepsilon\text{-Caprolactam (mass \%) in Reacted mixture)} \times (\text{Total mass (g) of Nylon 6, Solvent and Catalyst before Reaction})]/(\text{Mass (g) of Nylon 6 before Reaction}) \times 100$$

If no catalyst is used, then the mass of the catalyst is zero (g).

(Comparative examples 1 and 2)

Except that water was used in place of toluene and that the heating time was changed to 1 hour (Comparative example 1) and to 2 hours (Comparative example 2), a similar method was applied to perform depolymerization reactions, as in Example 1, to obtain the recovery percentages of ε-caprolactam. The pressure inside the reaction tube was 24.8 MPaG. The results are shown in Table 1. As a result, the recovery percentages of ε-caprolactam were found lower than Example 1. The interior of the reactor after the reaction lost the luster of stainless steel partly and became slightly black when it was visually confirmed.

(Examples 2-31)

Except that various conditions were changed as shown in Table 1, a similar method was applied to perform depolymerization reactions, as in Example 1. The pressure inside the reactor was 10 MPaG for Examples 6-8; 8.4 MPaG for Examples 13 and 14; 11.3 MPaG for Examples 15 and 16; 12.9 MPaG for Examples 17 and 18; 14.8 MPaG for Examples 19 and 20; 17.9 MPaG for Examples 21 and 22; and 20.7 MPaG for Example 28. The results are shown in Table 1. As a result, the recovery percentages of ε-caprolactam were found all extremely higher. The interior of the reactor after the reaction kept the luster of stainless steel when it was visually confirmed.

(Examples 32-35) (Reference examples)

An oligomer was used in place of nylon 6 to perform depolymerization reactions. The oligomer herein referred to is a residue resulted from removal of water from cleaning water used in hot water cleaning after the polymerization of ε-caprolactam and contains non-reacted ε-caprolactam or an oligomer of ε-caprolactam. A specific composition of the oligomer includes 22.5 mass % ε-caprolactam, 3.1 mass % aminocaproic acid, 16.6 mass % cyclic dimer, 21.1 mass % cyclic trimer, 13.4 mass % cyclic tetramer, 7.3 mass % cyclic pentamer, 1.8 mass % cyclic hexamer, 1.4 mass % cyclic heptamer, and 12.8 mass % others including chain oligomers. Except that the conditions were changed as shown in Table 1, a similar method was applied to perform depolymerization reactions, as in Example 1. The pressure inside the reactor was 14.8 MPaG for Examples 32-34, and 20.7 MPaG for Example 35. The results are shown in Table 1. As a result, the recovery percentages of ε-caprolactam were found extremely higher. The interior of the reactor after the reaction kept the luster of stainless steel when it was visually confirmed.

(Example 36)

Except that n-hexane (from Wako Pure Chemical Industries, Ltd., with 10 ppm of water) was used in place of toluene and that the condition was changed as shown in Table 1, a similar method was applied to perform a depolymerization reaction, as in Example 1. The pressure inside the reactor was 14.9 MPaG. The result is shown in Table 1. As a result, the recovery percentage of ε-caprolactam was found extremely higher. The interior of the reactor after the reaction kept the luster of stainless steel when it was visually confirmed.

[Table 1]

| | (1) | (2) | (3) | (4) | (5) | (6) | (7) | (8) |
|---|---|---|---|---|---|---|---|---|
| Example 1 | Nylon 6 | 0.3 | Toluene | 4.0 | 0.0 | 370 | 6 | 74.1 |
| Example 2 | Nylon 6 | 0.3 | Toluene | 3.99 | 0.01 | 370 | 2 | 72.3 |
| Example 3 | Nylon 6 | 0.3 | Toluene | 3.95 | 0.05 | 370 | 1 | 83.6 |
| Example 4 | Nylon 6 | 0.3 | Toluene | 3.95 | 0.05 | 370 | 2 | 91.0 |
| Example 5 | Nylon 6 | 0.3 | Toluene | 3.95 | 0.05 | 370 | 3 | 93.5 |
| Example 6 | Nylon 6 | 0.3 | Toluene | 3.9 | 0.1 | 370 | 1 | 89.9 |
| Example 7 | Nylon 6 | 0.3 | Toluene | 3.9 | 0.1 | 370 | 2 | 94.5 |
| Example 8 | Nylon 6 | 0.3 | Toluene | 3.9 | 0.1 | 370 | 3 | 92.9 |
| Example 9 | Nylon 6 | 0.6 | Toluene | 3.9 | 0.1 | 370 | 2 | 93.7 |
| Example 10 | Nylon 6 | 1.2 | Toluene | 3.9 | 0.1 | 370 | 2 | 82.0 |
| Example 11 | Nylon 6 | 0.3 | Toluene | 3.8 | 0.2 | 370 | 1 | 91.0 |
| Example 12 | Nylon 6 | 0.3 | Toluene | 3.8 | 0.2 | 370 | 2 | 92.0 |
| Example 13 | Nylon 6 | 0.3 | Toluene | 3.6 | 0.4 | 300 | 1 | 82.0 |
| Example 14 | Nylon 6 | 0.3 | Toluene | 3.6 | 0.4 | 300 | 2 | 91.5 |
| Example 15 | Nylon 6 | 0.3 | Toluene | 3.6 | 0.4 | 330 | 1 | 94.5 |
| Example 16 | Nylon 6 | 0.3 | Toluene | 3.6 | 0.4 | 330 | 2 | 93.1 |
| Example 17 | Nylon 6 | 0.3 | Toluene | 3.6 | 0.4 | 350 | 1 | 96.6 |
| Example 18 | Nylon 6 | 0.3 | Toluene | 3.6 | 0.4 | 350 | 2 | 94.3 |
| Example 19 | Nylon 6 | 0.3 | Toluene | 3.6 | 0.4 | 370 | 1 | 96.9 |
| Example 20 | Nylon 6 | 0.3 | Toluene | 3.6 | 0.4 | 370 | 2 | 93.1 |
| Example 21 | Nylon 6 | 0.3 | Toluene | 3.6 | 0.4 | 400 | 1 | 93.3 |
| Example 22 | Nylon 6 | 0.3 | Toluene | 3.6 | 0.4 | 400 | 2 | 94.6 |
| Example 23 | Nylon 6 | 1.2 | Toluene | 3.6 | 0.4 | 330 | 2 | 87.1 |
| Example 24 | Nylon 6 | 1.2 | Toluene | 3.6 | 0.4 | 350 | 2 | 89.0 |
| Example 25 | Nylon 6 | 1.2 | Toluene | 3.6 | 0.4 | 370 | 1 | 88.5 |
| Example 26 | Nylon 6 | 1.2 | Toluene | 3.6 | 0.4 | 370 | 2 | 94.3 |
| Example 27 | Nylon 6 | 2.0 | Toluene | 2.7 | 0.3 | 370 | 2 | 61.3 |
| Example 28 | Nylon 6 | 0.3 | Toluene | 3.2 | 0.8 | 370 | 1 | 94.4 |
| Example 29 | Nylon 6 | 1.2 | Toluene | 3.2 | 0.8 | 370 | 1 | 89.0 |
| Example 30 | Nylon 6 | 1.2 | Toluene | 3.2 | 0.8 | 370 | 2 | 87.3 |
| Example 31 | Nylon 6 | 1.2 | Toluene | 3.2 | 0.8 | 370 | 1 | 87.0 |
| Example 32 (Reference) | Oligomer | 1.26 | Toluene | 3.6 | 0.4 | 370 | 1 | 85.5 |
| Example 33 (Reference) | Oligomer | 1.26 | Toluene | 3.6 | 0.4 | 370 | 2 | 88.5 |
| Example 34 (Reference) | Oligomer | 1.26 | Toluene | 3.6 | 0.4 | 370 | 3 | 91.0 |

(continued)

|  | (1) | (2) | (3) | (4) | (5) | (6) | (7) | (8) |
|---|---|---|---|---|---|---|---|---|
| Example 35 (Reference) | Oligomer | 1.26 | Toluene | 3.2 | 0.8 | 370 | 2 | 81.7 |
| Example 36 | Nylon 6 | 1.2 | n-Hexane | 3.6 | 0.4 | 370 | 1 | 84.2 |
| Comparative example 1 | Nylon 6 | 0.3 | - | 0 | 4.0 | 370 | 1 | 67.7 |
| Comparative example 2 | Nylon 6 | 0.3 | - | 0 | 4.0 | 370 | 2 | 56.1 |
| (1) Type of Polyamide<br>(2) Polyamide (g)<br>(3) Type of Hydrocarbon Solvent<br>(4) Hydrocarbon Solvent (g)<br>(5) Water (g)<br>(6) Temperature (°C)<br>(7) Heating Time (hrs)<br>(8) Recovery percentage of Caprolactam (Mass % to Nylon) | | | | | | | | |

Next, types of the hydrocarbon solvent were changed as shown in Table 2 to perform depolymerization of nylon 6. Except that hydrocarbon solvents other than toluene were used and the heating was performed for 3 hours in addition to 1 and 2 hours, a similar method was applied to perform depolymerization reactions, as in Examples 19 and 20 in which the ratio of water relative to the total mass of the hydrocarbon solvent and water is 10 mass %. The hydrocarbon solvents used other than toluene include n-hexane, cyclohexane, n-dodecane, n-heptane, n-octane, cyclooctane, n-tridecane, n-tetradecane, n-pentadecane, liquid paraffin (special grade from Wako Pure Chemical Industries, Ltd.), and tetrahydro naphthalene (first grade from Wako Pure Chemical Industries, Ltd.). The pressure inside the reactor of Example 64 was 5.1 MPaG. As a result, the recovery percentages of ε-caprolactam were found extremely higher as shown in Table 2 even when hydrocarbon solvents other than toluene were used. The interior of the reactor after the reaction kept the luster of stainless steel when it was visually confirmed.

[Table 2]

|  | (1) | (2) | (3) | (4) | (5) | (6) | (7) | (8) |
|---|---|---|---|---|---|---|---|---|
| Example 19 | Nylon 6 | 0.3 | 10% Water /Toluene | 3.6 | 0.4 | 370 | 1 | 96.93 |
| Example 20 | Nylon 6 | 0.3 | 10% Water /Toluene | 3.6 | 0.4 | 370 | 2 | 93.14 |
| Example 37 | Nylon 6 | 0.3 | 10% Water /n-Hexane | 3.6 | 0.4 | 370 | 1 | 91.10 |
| Example 38 | Nylon 6 | 0.3 | 10% Water /n-Hexane | 3.6 | 0.4 | 370 | 2 | 91.16 |
| Example 39 | Nylon 6 | 0.3 | 10% Water /n-Hexane | 3.6 | 0.4 | 370 | 3 | 92.61 |
| Example 40 | Nylon 6 | 0.3 | 10% Water /CycloHexane | 3.6 | 0.4 | 370 | 1 | 90.46 |
| Example 41 | Nylon 6 | 0.3 | 10% Water /CycloHexane | 3.6 | 0.4 | 370 | 2 | 91.19 |
| Example 42 | Nylon 6 | 0.3 | 10% Water /CycloHexane | 3.6 | 0.4 | 370 | 3 | 90.24 |
| Example 43 | Nylon 6 | 0.3 | 10% Water /n-Dodecane | 3.6 | 0.4 | 370 | 1 | 95.73 |
| Example 44 | Nylon 6 | 0.3 | 10% Water /n-Dodecane | 3.6 | 0.4 | 370 | 2 | 95.23 |
| Example 45 | Nylon 6 | 0.3 | 10% Water /n-Dodecane | 3.6. | 0.4 | 370 | 3 | 94.95 |
| Example 46 | Nylon 6 | 0.3 | 10% Water /n-Heptane | 3.6 | 0.4 | 370 | 1 | 91.88 |
| Example 47 | Nylon 6 | 0.3 | 10% Water /n-Heptane | 3.6 | 0.4 | 370 | 2 | 92.01 |
| Example 48 | Nylon 6 | 0.3 | 10% Water /n-Heptane | 3.6 | 0.4 | 370 | 3 | 92.05 |
| Example 49 | Nylon 6 | 0.3 | 10% Water /n-Octane | 3.6 | 0.4 | 370 | 1 | 89.70 |
| Example 50 | Nylon 6 | 0.3 | 10% Water /n-Octane | 3.6 | 0.4 | 370 | 2 | 91.32 |
| Example 51 | Nylon 6 | 0.3 | 10% Water /n-Octane | 3.6 | 0.4 | 370 | 3 | 93.98 |

(continued)

|  | (1) | (2) | (3) |  | (4) | (5) | (6) | (7) | (8) |
|---|---|---|---|---|---|---|---|---|---|
| Example 52 | Nylon 6 | 0.3 | 10% Water /CycloOctane |  | 3.6 | 0.4 | 370 | 1 | 92.54 |
| Example 53 | Nylon 6 | 0.3 | 10% Water /CycloOctane |  | 3.6 | 0.4 | 370 | 2 | 92.13 |
| Example 54 | Nylon 6 | 0.3 | 10% Water /CycloOctane |  | 3.6 | 0.4 | 370 | 3 | 91.93 |
| Example 55 | Nylon 6 | 0.3 | 10% Water /n-Tridecane |  | 3.6 | 0.4 | 370 | 1 | 93.67 |
| Example 56 | Nylon 6 | 0.3 | 10% Water ln-Tridecane |  | 3.6 | 0.4 | 370 | 2 | 92.17 |
| Example 57 | Nylon 6 | 0.3 | 10% Water /n-Tridecane |  | 3.6 | 0.4 | 370 | 3 | 92.67 |
| Example 58 | Nylon 6 | 0.3 | 10% Water /n-Tetradecane |  | 3.6 | 0.4 | 370 | 1 | 92.96 |
| Example 59 | Nylon 6 | 0.3 | 10% Water /n-Tetradecane |  | 3.6 | 0.4 | 370 | 2 | 93.19 |
| Example 60 | Nylon 6 | 0.3 | 10% Water /n-Tetradecane |  | 3.6 | 0.4 | 370 | 3 | 93.27 |
| Example 61 | Nylon 6 | 0.3 | 10% Water /n-Pentadecane |  | 3.6 | 0.4 | 370 | 1 | 93.16 |
| Example 62 | Nylon 6 | 0.3 | 10% Water /n-Pentadecane |  | 3.6 | 0.4 | 370 | 2 | 94.11 |
| Example 63 | Nylon 6 | 0.3 | 10% Water /n-Pentadecane |  | 3.6 | 0.4 | 370 | 3 | 93.78 |
| Example 64 | Nylon 6 | 0.3 | 10% Water /Liquid paraffin |  | 3.6 | 0.4 | 370 | 1 | 73.50 |
| Example 65 | Nylon 6 | 0.3 | 10% Water /Liquid paraffin |  | 3.6 | 0.4 | 370 | 2 | 81.24 |
| Example 66 | Nylon 6 | 0.3 | 10% Water /Liquid paraffin |  | 3.6 | 0.4 | 370 | 3 | 87.21 |
| Example 67 | Nylon 6 | 0.3 | 10% Water /Tetrahydro naphthalene |  | 3.6 | 0.4 | 370 | 1 | 90.50 |
| Example 68 | Nylon 6 | 0.3 | 10% Water /Tetrahydro naphthalene |  | 3.6 | 0.4 | 370 | 2 | 91.50 |
| Example 69 | Nylon 6 | 0.3 | 10% Water /Tetrahydro naphthalene |  | 3.6 | 0.4 | 370 | 3 | 91.62 |

(1) Type of Polyamide
(2) Polyamide (g)
(3) Type of Solvent
(4) Hydrocarbon Solvent (g)
(5) Water (g)
(6) Temperature (°C)
(7) Heating Time (hrs)
(8) Recovery percentage of Caprolactam (Mass % to Nylon)

(Examples 70-74)

Next, the ratio of water relative to the total mass of the hydrocarbon solvent and water was changed to perform depolymerization of nylon 6 as shown in Table 3. Except that the ratio of water relative to the total mass of the hydrocarbon solvent and water was changed, a similar method was applied to perform depolymerization reactions, as in Examples 3, 6, 11, 19 and 28 in which heating was performed at 370 °C for 1 hour. As a result, the recovery percentages of ε-caprolactam were found extremely higher as shown in Table 3 when the ratio of water relative to the total mass of the hydrocarbon solvent and water is above 0 and not more than 30 mass %. Further, the recovery percentages of ε-caprolactam were found particularly higher when the ratio of water relative to the total mass of the hydrocarbon solvent and water is not less than 1 mass % and not more than 20 mass %. The interior of the reactor after the reaction kept the luster of stainless steel when it was visually confirmed for Examples 3, 6, 11, 19, 28 and 70 in which the ratio of water relative to the total mass of the hydrocarbon solvent and water is not more than 30 mass %.
[Table 3]

|  | (1) | (2) | (3) | (4) | (5) | (6) | (7) | (8) |
|---|---|---|---|---|---|---|---|---|
| Example 3 | Nylon 6 | 0.3 | 1.25% Water /Toluene | 3.95 | 0.05 | 370 | 1 | 83.64 |
| Example 6 | Nylon 6 | 0.3 | 2.5% Water /toluene | 3.9 | 0.1 | 370 | 1 | 89.90 |

(continued)

|  | (1) | (2) | (3) | (4) | (5) | (6) | (7) | (8) |
|---|---|---|---|---|---|---|---|---|
| Example 11 | Nylon 6 | 0.3 | 5% Water /Toluene | 3.8 | 0.2 | 370 | 1 | 90.98 |
| Example 19 | Nylon 6 | 0.3 | 10% Water /Toluene | 3.6 | 0.4 | 370 | 1 | 96.93 |
| Example 28 | Nylon 6 | 0.3 | 20% Water /Toluene | 3.2 | 0.8 | 370 | 1 | 94.42 |
| Example 70 | Nylon 6 | 0.3 | 30% Water /Toluene | 2.8 | 1.2 | 370 | 1 | 86.46 |
| Example 71 | Nylon 6 | 0.3 | 40% Water /Toluene | 2.4 | 1.6 | 370 | 1 | 86.13 |
| Example 72 | Nylon 6 | 0.3 | 50% Water /Toluene | 2.0 | 2.0 | 370 | 1 | 84.67 |
| Example 73 | Nylon 6 | 0.3 | 60% Water /Toluene | 1.6 | 2.4 | 370 | 1 | 81.81 |
| Example 74 | Nylon 6 | 0.3 | 70% Water /Toluene | 1.2 | 2.8 | 370 | 1 | 73.24 |
| (1) Type of Polyamide<br>(2) Polyamide (g)<br>(3) Type of Solvent<br>(4) Hydrocarbon Solvent (g)<br>(5) Water (g)<br>(6) Temperature (°C)<br>(7) Heating Time (hrs)<br>(8) Recovery percentage of Caprolactam (Mass % to Nylon) | | | | | | | | |

(Examples 75-77)

Next, the depolymerization reaction temperature was changed to perform depolymerization of nylon 6 as shown in Table 4. Except that the depolymerization reaction temperature was changed, a similar method was applied to perform depolymerization reactions, as in Examples 13, 15, 17, 19 and 21 in which the ratio of water relative to the total mass of the hydrocarbon solvent and water is 10 mass %. As a result, the recovery percentages of ε-caprolactam were found extremely higher even when the depolymerization reaction temperature was changed. Further, the recovery percentages of ε-caprolactam were found particularly higher when the depolymerization reaction temperature is not lower than 300 °C and not higher than 420 °C. The recovery percentages of ε-caprolactam were found extremely higher when the depolymerization reaction temperature is not lower than 350 °C and not higher than 370 °C. The interior of the reactor after the reaction kept the luster of stainless steel when it was visually confirmed.

[Table 4]

|  | (1) | (2) | (3) | (4) | (5) | (6) | (7) | (8) |
|---|---|---|---|---|---|---|---|---|
| Example 75 | Nylon 6 | 0.3 | 10% Water /Toluene | 3.6 | 0.4 | 280 | 1 | 72.18 |
| Example 13 | Nylon 6 | 0.3 | 10% Water /Toluene | 3.6 | 0.4 | 300 | 1 | 82.04 |
| Example 15 | Nylon 6 | 0.3 | 10% Water /Toluene | 3.6 | 0.4 | 330 | 1 | 94.54 |
| Example 17 | Nylon 6 | 0.3 | 10% Water /Toluene | 3.6 | 0.4 | 350 | 1 | 96.60 |
| Example 19 | Nylon 6 | 0.3 | 10% Water /Toluene | 3.6 | 0.4 | 370 | 1 | 96.93 |
| Example 21 | Nylon 6 | 0.3 | 10% Water /Toluene | 3.6 | 0.4 | 400 | 1 | 93.27 |
| Example 76 | Nylon 6 | 0.3 | 10% Water /Toluene | 3.6 | 0.4 | 420 | 1 | 89.66 |

(continued)

|  | (1) | (2) | (3) | (4) | (5) | (6) | (7) | (8) |
|---|---|---|---|---|---|---|---|---|
| Example 77 | Nylon 6 | 0.3 | 10% Water /Toluene | 3.6 | 0.4 | 450 | 1 | 71.69 |

(1) Type of Polyamide
(2) Polyamide (g)
(3) Type of Solvent
(4) Hydrocarbon Solvent (g)
(5) Water (g)
(6) Temperature (°C)
(7) Heating Time (hrs)
(8) Recovery percentage of Caprolactam (Mass % to Nylon)

INDUSTRIAL AVAILABILITY

The present invention is available in recovering caprolactam at a higher recovery percentage by depolymerizing Nylon 6 products.

**Claims**

1. Nylon 6 depolymerizing method for depolymerizing nylon 6 to obtain caprolactam, comprising performing a depolymerization reaction in the presence of a hydrocarbon solvent and water without using a catalyst at a temperature not lower than 300 °C and not higher than 420 °C.

2. The nylon 6 depolymerizing method according to claim 1, wherein the ratio of water relative to the total mass of the hydrocarbon solvent and water is not more than 30 mass %.

3. The nylon 6 depolymerizing method according to claims 1 or 2, wherein the hydrocarbon solvent is toluene.

4. Caprolactam producing method, comprising subjecting nylon 6 to a depolymerization reaction in the presence of a hydrocarbon solvent and water without using a catalyst at a temperature not lower than 300 °C and not higher than 420 °Cto obtain the caprolactam.

5. The caprolactam producing method according to claim 4, wherein the ratio of water relative to the total mass of the hydrocarbon solvent and water is not more than 30 mass %.

6. The caprolactam producing method according to claims 4 or 5, wherein the hydrocarbon solvent is toluene.

**Patentansprüche**

1. Nylon 6 depolymerisierendes Verfahren zum Depolymerisieren von Nylon 6 zum Erhalt von Caprolactam, umfassend das Durchführen einer Depolymerisationsreaktion in der Gegenwart eines Kohlenwasserstofflösungsmittels und von Wasser ohne die Verwendung eines Katalysators bei einer Temperatur von nicht niedriger als 300°C und nicht höher als 420°C.

2. Nylon 6 depolymerisierendes Verfahren nach Anspruch 1, wobei das Verhältnis von Wasser in Bezug auf die Gesamtmasse des Kohlenwasserstofflösungsmittels und Wassers nicht mehr als 30 Massen-% beträgt.

3. Nylon 6 depolymerisierendes Verfahren nach den Ansprüchen 1 oder 2, wobei das Kohlenwasserstofflösungsmittel Toluol ist.

4. Caprolactam herstellendes Verfahren, umfassend das Unterziehen von Nylon 6 einer Depolymerisationsreaktion in der Gegenwart eines Kohlenwasserstofflösungsmittels und von Wasser ohne die Verwendung eines Katalysators bei einer Temperatur von nicht niedriger als 300°C und nicht höher als 420°C zum Erhalt des Caprolactams.

**5.** Caprolactam herstellendes Verfahren nach Anspruch 4, wobei das Verhältnis von Wasser in Bezug auf die Gesamtmasse des Kohlenwasserstoff lösungsmittels und Wassers nicht mehr als 30 Massen-% beträgt.

**6.** Caprolactam herstellendes Verfahren nach den Ansprüchen 4 oder 5, wobei das Kohlenwasserstofflösungsmittel Toluol ist.


**Revendications**

**1.** Procédé de dépolymérisation du nylon 6 pour dépolymériser le nylon 6 afin d'obtenir du caprolactame, comprenant l'exécution d'une réaction de dépolymérisation en présence d'un solvant hydrocarboné et d'eau sans utiliser de catalyseur à une température non inférieure à 300°C et non supérieure à 420°C.

**2.** Le procédé de dépolymérisation du nylon 6 selon la revendication 1, dans lequel le rapport de l'eau par rapport à la masse totale du solvant hydrocarboné avec l'eau n'est pas supérieur à 30 % en masse.

**3.** Le procédé de dépolymérisation du nylon 6 selon les revendications 1 ou 2, dans lequel le solvant hydrocarboné est le toluène.

**4.** Procédé de production de caprolactame, comprenant la soumission du nylon 6 à une réaction de dépolymérisation en présence d'un solvant hydrocarboné et d'eau sans utiliser de catalyseur à une température non inférieure à 300°C et non supérieure à 420°C pour obtenir le caprolactame.

**5.** Le procédé de production de caprolactame selon la revendication 4, dans lequel le rapport de l'eau par rapport à la masse totale du solvant hydrocarboné avec l'eau n'est pas supérieur à 30 % en masse.

**6.** Le procédé de production de caprolactame selon la revendication 4 ou 5, dans lequel le solvant hydrocarboné est le toluène.

**REFERENCES CITED IN THE DESCRIPTION**

**Patent documents cited in the description**

- JP 8301843 A